# EUROPÄISCHE PATENTANMELDUNG

(11) **EP 1 283 420 A1**
(43) Veröffentlichungstag der Anmeldung: **12.02.2003**
(21) Anmeldenummer: 01119089.9
(22) Anmeldetag: 08.08.2001
(51) Int. Cl.: G01N 33/18, G01N 27/416

(54) **Verfahren und Vorrichtung zur Bestimmung einer Kohlenwasserstoffkonzentration in Wasser**

(71) Anmelder: Loggion AG, 24148 Kiel (DE)
(72) Erfinder: Holstermann, Dipl-Phys. Gregor, 24119 Kronshagen (DE); Lukas, Dr. Dirk, 24105 Kiel (DE); Mörz, Dipl-Geol. Tobias, 24235 Laboe (DE); Weppner, Prof. Dr. Werner, 24226 Heikendorf (DE)
(74) Vertreter: Patentanwälte Rehberg + Hüppe

(57) **Zusammenfassung**

Zur Bestimmung einer Kohlenwasserstoffkonzentration, insbesondere einer Methankonzentration, in Wasser (14), wird die von der Kohlenwasserstoffkonzentration abhängige Leitfähigkeit über eine Protonenaustauschmembran (PEM) (2) bestimmt.

## Beschreibung

Die Erfindung bezieht sich auf ein Verfahren zur Bestimmung einer Kohlenwasserstoffkonzentration, insbesondere einer Methankonzentration, in Wasser, wobei eine von der Kohlenwasserstoffkonzentration abhängige Leitfähigkeit bestimmt wird. Weiterhin bezieht sich die Erfindung auf eine Vorrichtung zur Bestimmung einer Kohlenwasserstoffkonzentration, insbesondere einer Methankonzentration, in Wasser, mit einer Messanordnung, deren Leitfähigkeit von der Kohlenwasserstoffkonzentration abhängig ist.

Verfahren und Vorrichtungen zur Bestimmung der Konzentration von in Wasser gelösten Kohlenwasserstoffen, insbesondere von Methankonzentrationen, werden beispielsweise von den marinen Geowissenschaften, der sich mit der Erdölexploration beschäftigenden Off-shore-Industrie und der Wasserwirtschaft nachgefragt. Eine Anforderung dabei ist, dass die Methankonzentrationen am Ort ihres Auftretens (in situ) innerhalb kurzer Zeiträume bestimmt werden können und die dabei anfallenden Messsignale entweder elektronisch gespeichert oder weitergeleitet werden können. Darüberhinaus muss eine Funktionsfähigkeit im Bereich niedriger Temperaturen von typischerweise -2 bis 30 °C und hohen Drücken von 200 bar und mehr gegeben sein. Gleichzeitig sind eine hohe Lebensdauer der Vorrichtung sowie ein geringer Wartungs- und Energiebedarf von Interesse.

Ein Verfahren und eine Vorrichtung der eingangs beschriebenen Art sind aus Nikolai Franz: "Analyse der Wirkung von Umwelteinflüssen und Drift auf das Messsignal von Unterwassermethansensoren", Diplomarbeit, Fachhochschule Lübeck, 1999, bekannt. Der dort beschriebene Unterwassermethansensor bestimmt die Methankonzentration nicht direkt in der Wasserphase. Vielmehr ist der Wasserphase eine für flüssiges Wasser undurchlässige Membran zugekehrt, durch die nur Wasserdampf und Gase hindurchtreten können. Die Membran wird gegen den einwirkenden Wasserdruck durch eine Sintermetallscheibe abgestützt. Hinter der Sintermetallscheibe ist ein Gasraum angeordnet, in dem sich nach dem Henryschen Gesetz eine mit der Wasserphase im Gleichgewicht stehende Methankonzentration einstellt. In dem Gasraum ist eine Messanordnung vorgesehen, die einen Halbleiter auf Zinndioxidbasis aufweist. Die elektrische Leitfähigkeit diese Halbleiters ist von der Methankonzentration im Gasraum abhängig. Konkret steigt die Leitfähigkeit des Halbleiters mit steigender Konzentration des Methans an. Hieraus läßt sich die Methankonzentration in der Wasserphase indirekt bestimmen. Dabei muss aber berücksichtigt werden, dass die Leitfähigkeit des Halbleiters neben einer für Halbleiter typischen Temperaturabhängigkeit auch eine Querempfindlichkeit zur Feuchtigkeit in dem Gasraum aufweist. Daher befinden sich Feuchte- und Temperaturfühler in dem Gasraum, um die absolute Feuchte in dem Gasraum bestimmen und Ihren Einfluss kompensieren zu können.

Die bekannte Vorrichtung zur Bestimmung einer Methankonzentration und das ihr zugrundeliegende Verfahren sind träge, weil sich erst eine der Wasserphase entsprechende Methankonzentration in dem Gasraum einstellen muss, um die Methankonzentration in der Wasserphase indirekt bestimmen zu können. Durch die Sintermetallscheibe zur Abstützung des Wasserdrucks auf die Membran wird die Einstellung eines Gleichgewichts der Methankonzentrationen in der Wasserphase und dem Gasraum weiter verzögert. In der Anwendung der bekannten Vorrichtung ist überdies festgestellt worden, dass sich die Permeabilität der Membran über die Einsatzdauer der Vorrichtung verändert. So sind die Eigenschaften der Vorrichtung, d. h. das von ihr primär abgegebene Messsignal ganz erheblich von der Vorgeschichte der Verwendung der Vorrichtung abhängig, wobei eine maximale Empfindlichkeit für die Methankonzentrationsbestimmung beispielsweise eine vorhergehende Konditionierung des Halbleiters in definierter Umgebung erfordert. Darüberhinaus zeigt sich eine Druckabhängigkeit der als Maß für die Methankonzentrationen gemessenen Leitfähigkeiten.

Aus VDI-Technologie Zentrum: "Festkörper-Ionik", 1999, ist es bekannt, Festelektrolysezellen zur Messung von unbekannten Sauerstoffkonzentrationen zu verwenden, indem eine sich an einer Festelektrolysezelle einstellende Spannung gemessen wird. Dabei ist Sauerstoff ein Reaktionspartner, einer die gemessene Spannung erzeugenden Reaktion. Weiterhin ist es aus dieser Druckschrift bekannt, dass auch die Konzentrationen von mit dem Sauerstoff im theromdynamischen Gleichgewicht stehenden Substanzen indirekt durch die Messung der Spannung an der Festelektrolysezelle als Maß für die Sauerstoffkonzentration bestimmt werden können. Unter einer Festelektrolysezelle ist dabei eine Brennstoffzelle mit einem Festelektrolyt als Mittelschicht zu verstehen, beispielsweise eine sogenannte PEM-Brennstoffzelle.

Der Erfindung liegt die Aufgabe zugrunde, ein Verfahren und eine Vorrichtung der eingangs beschriebenen Art aufzuzeigen, die unter den besonderen Anforderungen an maritime Anwendungen in der Lage sind, auf schwankende Kohlenwasserstoffkonzentrationen, insbesondere Methankonzentrationen, zu reagieren.

Bei dem Verfahren der eingangs beschriebenen Art wird die Aufgabe der Erfindung dadurch gelöst, dass die Leitfähigkeit, die von der Kohlenwasserstoffkonzentration abhängig ist, über eine Protonenaustauschmembran (PEM) bestimmt wird.

Der Erfindung liegt die Erkenntnis zugrunde, dass Kohlenwasserstoffkonzentrationen in Wasser und insbesondere Konzentrationen kurzkettiger Kohlenwasserstoffe, für die Methan hier stellvertretend steht, in Wasser Einfluss auf die Leitfähigkeit einer Protonenaustauschmembran haben. D. h., der auf Protonenaustausch basierende Strom über eine PEM variiert bei fester Spannung über die PEM beispielsweise mit der Methankonzentration in an die PEM angrenzendem Wasser.

Hintergrund dieses Effekts dürfte eine Art kovalente Anbindung der Kohlenwasserstoffmoleküle an die PEM sein, wobei das über die PEM anliegende elektrische Feld aufgrund der die Protonenleitung verursachenden Spannung allenfalls von untergeordneter Bedeutung ist. Die an die PEM angelagerten Kohlenwasserstoffmoleküle reduzieren die Leitfähigkeit der PEM durch Behinderung des Protonentransports, wobei das Maß der Reduktion von der Kohlenwasserstoffkonzentration in dem an die PEM angrenzenden Wasser direkt abhängig ist. Weiterhin ist die Anbindung der Kohlenwasserstoffmoleküle an die Membran reversibel und es stellt sich schnell ein thermodynamisches Gleichgewicht zwischen der Kohlenwasserstoffkonzentration in dem Wasser und den an die PEM angebundenen Kohlenwasserstoffmolekülen ein. So wird problemlos die gewünschte Dynamik des Verfahrens bei der Kohlenwasserstoffkonzentrationsbestimmung erreicht. Es kann aber auch die Kinetik der Änderung der Leitfähigkeit der PEM in die Auswertung einbezogen werden. Hierdurch können einerseits Messergebnisse noch schneller erhalten und andererseits die Konzentrationen verschiedener Kohlenwasserstoffkonzentrationen gegeneinander diskriminiert werden. Aufgrund des von dem elektrischen Feld über die PEM weitgehend unabhängigen Vorgangs, der der Kohlenwasserstoffkonzentrationsbestimmung zugrundeliegt, kommt es für das neue Verfahren nicht darauf an, auf welcher Seite der PEM das Wasser mit der interessierenden Methankonzentration angeordnet wird. Grundsätzlich ist auch denkbar, dieses Wasser auf beiden Seiten der PEM vorzusehen.

Zugunsten definierter Messbedingungen ist es aber bevorzugt, auf der einen Seite der PEM das Wasser, in dem die Kohlenwasserstoffkonzentration zu bestimmen ist, und auf der anderen Seite der PEM eine Referenzsubstanz anzuordnen. Bei der Referenzsubstanz kann es sich um eine wässrige Referenzflüssigkeit aber beispielsweise auch um eine flüssige oder feste Wasserstoffquelle, beispielsweise eine flüssige Wasserstofflösung oder einen festen mit Wasserstoff beladenen Metallhydridspeicher handeln. Die wässrigen Referenzflüssigkeit kann destilliertes Wasser oder eine andere Flüssigkeit definierter Zusammensetzung sein. In jedem Fall ist die Art und Zusammensetzung der Referenzsubstanz bei der Auswertung der gemessenen Werte der Leitfähigkeit über die PEM zu berücksichtigen.

Es wurde zwar bereits ausgeführt, dass sich das Gleichgewicht zwischen den an die Membran angelagerten Methanmolekülen und der Methankonzentration im Wasser schnell einstellt. Um aber auf schwankende Methankonzentrationen in der Umgebung zügig anzusprechen, sollte zusätzlich das Wasser zu einer Strömung über die Fläche der jeweiligen Seite der PEM gezwungen werden, um Wasser mit der interessierenden Methankonzentration möglichst schnell und dicht an die PEM heranzubringen. Auch auf der Referenzseite ist eine Zirkulation einer flüssigen Referenzsubstanz sinnvoll, um etwaige Veränderungen der Referenzsubstanz über die PEM hinweg schnell auszugleichen.

Vorzugsweise wird die Leitfähigkeit über die PEM so gemessen, dass eine feste Spannung über die PEM angelegt wird und dass der bei dieser Spannung fließende Strom über die PEM gemessen wird. Die Größe der Spannung wird so gewählt, dass bei einer wässrigen Referenzsubstanz gerade eine Elektrolyse von Wasser über die PEM hinweg erfolgen kann. Viel höhere Spannungen sind eher nachteilig.

Wenn eine Gleichspannung über die PEM angelegt wird, kann das positive oder das negative Potential auf der Seite der PEM angelegt werden, auf der das Wasser, in dem die Kohlenwasserstoffkonzentration zu bestimmen ist, angeordnet werden. Wie bereits mehrfach erwähnt, wird der dem erfindungsgemäßen Verfahren zugrundeliegende Effekt der Behinderung der Leitfähigkeit durch an die PEM angelagerte Methanmoleküle unabhängig von dem über die PEM von außen angelegten elektrischen Feld beobachtet. So kann die Anordnung des Wassers, in dem die Kohlenwasserstoffkonzentration zu bestimmen ist, von anderen Aspekten abhängig gemacht werden. Beispielsweise kann das Wasser, in dem die Kohlenwasserstoffkonzentration zu bestimmen ist, auf die H2-Seite der PEM verlegt werden, um dort gezielt Oxidationsprozesse aufgrund des auf der Sauerstoffseite freigesetzten Sauerstoffs oder Reaktionen von Katalysator- und Elektrodenmaterialien mit nascierendem Chlor, das bei der Hydrolyse von Salzwasser anfällt, oder andere Effekte zu verhindern.

Grundsätzlich ist es aber auch denkbar, eine Wechselspannung über die PEM anzulegen, und die sich dabei ergebene Leitfähigkeit der PEM zu bestimmen. Allerdings muss dabei der Entstehung gefährlicher Konzentrationen an Knallgas vorgebeugt werden. Als günstig und ungefährlich erweist es sich hingegen, unipolare Spannungspulse an die Membran anzulegen, und dabei nicht nur die Endwerte der Leitfähigkeit über die PEM sondern auch die Kinetik der Leitfähigkeit zu beobachten und auszuwerten. Außerdem kann die PEM durch kurze Pulse in Gegenpolung einer Art Rekonditionierung unterzogen werden, ohne dass hierdurch schon relevante Mengen an Knallgas entstehen könnten.

Um die Zugänglichkeit der PEM für das Wasser, in dem die Kohlenwasserstoffkonzentration zu bestimmen ist, zu erhöhen, kann zumindest auf seiner Seite der PEM ein massiver Elektrodenrahmen im Randbereich der PEM angeordnet werden, der mit einer leitenden Katalysatorschicht auf der PEM kontaktiert wird. Mit anderen Worten wird nicht versucht, die PEM flächig bzw. quasiflächig mit der massiven Elektrode zu kontaktieren. Zugunsten der Zugänglichkeit der PEM für das zu untersuchende Wasser wird vielmehr die Leitfähigkeit einer leitenden Katalysatorschicht, die flächig über die PEM verteilt ist, ausgenutzt. Bei der leitenden Katalysatorschicht handelt es sich üblicherweise um Platin in der Form von Platinschwarz.

Wenn das Wasser, in dem die Kohlenwasserstoffkonzentration zu bestimmen ist, Salzwasser ist, besteht die grundsätzliche Gefahr, dass höherwertige Ionen aus dem Salzwasser die PEM zusetzen. Um diese Gefahr zu beseitigen, kann auf der Seite der PEM, auf der das Wasser, in dem die Kohlenwasserstoffkonzentration zu bestimmen ist, angeordnet wird, eine Silikonmembran vor der PEM angeordnet werden. Die Silikonmembran liegt dabei über der Oberfläche der PEM und einem dort angeordnetem Katalysator sowie der angrenzenden Elektrode. Durch eine Zirkulation des Wassers auf der Rückseite der Membran und die auftretenden osmotischen Effekte kann trotz der Silikonmembran ein schnelles Anpassen des Messsignals an schwankenden Methankonzentrationen im Wasser beobachtet werden. Dabei ist zu berücksichtigen, dass die bei dem erfindungsgemäßen Verfahren eingesetzte Membran keine Wasserphase von einer Gasphase abtrennt, sondern zwischen zwei Flüssigphasen angeordnet ist. Sie muss auch nicht gegen den auf der Seite des Wassers, dessen Kohlenwasserstoffkonzentration zu bestimmen ist, herrschenden statischen Druck durch eine die Wege verlängernde Sinterplatte oder dgl. abgestützt werden. Vielmehr folgt die Abstützung der Silikonmembran wie auch der PEM durch einen Druckausgleich zwischen den beiden Seiten der PEM. Mit anderen Worten ist ein isostatischer Aufbau gegeben, bei dem der Druck auf beiden Seiten der PEM bzw. der Silikonmembran gleich ist.

Bei dem erfindungsgemäßen Verfahren ist festzustellen, dass die gemessenen Leitfähigkeiten der PEM von der Temperatur und von dem Sauerstoffgehalt des Wassers abhängen können. Um diese Effekt zu kompensieren ,ist es daher sinnvoll, entweder die Temperatur, den Sauerstoffgehalt oder auch andere relevante Parameter des Wassers neben der zu bestimmenden Kohlenwasserstoffkonzentration auf vorgegebene Werte einzustellen oder zumindest zu bestimmen, um ggf. eine messwertseitige Kompensation vorzunehmen.

Eine Vorrichtung der eingangs beschriebenen Art ist erfindungsgemäß dadurch gekennzeichnet, dass die Messanordnung eine Protonenaustauschmembran (PEM) aufweist. Diese Protonenaustauschmembran ist vorzugsweise eine Polymerelektrolytmembran. Geeignet ist beispielsweise ein unter der Marke Nafion von der Firma Du Pont verfügbares Produkt.

Auf der einen Seite der PEM kann ein Raum für das Wasser, in dem die Kohlenwasserstoffkonzentration zu bestimmen ist, angeordnet sein, während auf der anderen Seite der PEM eine Referenzsubstanz angeordnet ist, bei der es sich um eine insbesondere wässrige Referenzflüssigkeit aber beispielsweise auch um eine flüssige oder feste Referenzsubstanz in Form einer Wasserstoffquelle handeln kann.

Dabei können der Raum für das Wasser und/oder ein Raum für eine wässrige Referenzflüssigkeit ein über die Fläche der jeweiligen Seite der PEM führendes Leitungssystem aufweisen, wobei eine Pumpe vorgesehen ist, die das Wasser bzw. die Referenzsubstanz durch das Leitungssystem hindurchpumpt. Das Leitungssystem ermöglicht die Zirkulation der jeweiligen Flüssigkeit über die PEM. Je kleiner dabei die laminare Grenzschicht an der PEM wird, desto schneller reagiert die Vorrichtung durch Leitfähigkeitsänderungen der PEM auf schwankende Methankonzentrationen.

Für die eigentliche Messung der Leitfähigkeit über die PEM kann eine Spannungsquelle vorgesehen sein, die eine feste Spannung über die PEM abgibt, wobei ein Amperemeter vorgesehen ist, das den bei dieser Spannung fließenden Strom über die PEM misst.

Bei der Spannungsquelle kann es sich um eine Gleichspannungsquelle handeln, die eine Gleichspannung über die PEM abgibt, wobei das positive oder das negative Potential auf der Seite der PEM anliegt, auf der der Raum für das Wasser, in dem die Kohlenwasserstoffkonzentration zu bestimmen ist, angeordnet ist.

Bei der Spannungsquelle kann es sich aber auch um eine Wechselspannungsquelle handeln, die eine Wechselspannung über die PEM abgibt.

Besonders bevorzugt ist eine Spannungsquelle, die unipolare Spannungspulse über die PEM abgibt, wobei ein Amperemeter zur Erfassung des resultierenden Stroms den Verlauf der Stromstärke über der Zeit auflöst, um auch dynamische Effekte erfassen und auswerten zu können.

Die Spannungspulse kann darüberhinaus zur Abgabe von kurzen gegenpoligen Rekonditionierungspulsen für die PEM vorgesehen sein.

In einer bevorzugten Ausführungsform ist auf der Seite der PEM, auf der der Raum für das Wasser, in dem die Kohlenwasserstoffkonzentration zu bestimmen ist, angeordnet ist, ein massiver Elektrodenrahmen im Randbereich der PEM angeordnet und mit einer leitenden Katalysatorschicht auf der PEM kontaktiert.

Zwischen dem Raum für das Wasser, in dem die Kohlenwasserstoffkonzentration zu bestimmen ist, und der PEM, ist in einer bevorzugten Ausführungsform zur Bestimmung von Kohlenwasserstoffkonzentrationen in Salzwasser eine Silikonmembran angeordnet.

Zur Unterdrückung bzw. zur Kompensation von Querempfindlichkeiten sind weiterhin vorzugsweise Konditionier- und/oder Messeinrichtungen vorgesehen, um die Parameter des Wassers, die neben der zu bestimmenden Kohlenwasserstoffkonzentration die Leitfähigkeit der PEM beeinflussen können, auf vorgegebene Werte einzustellen und/oder zu bestimmen. Hierbei handelt es sich insbesondere um Temperatur- und Sauerstoffkonzentrationsfühler.

Die Erfindung wird im folgenden anhand von Ausführungsbeispielen näher erläutert und beschrieben, dabei zeigt
- Fig. 1: eine Anordnung zur Durchführung des Verfahrens zur Bestimmung von Kohlenwasserstoffkonzentrationen in Wasser,
- Fig. 2: den Aufbau einer Festelektrolytzelle als Bestandteil der Anordnung gemäß Fig. 1,
- Fig. 3: eine Aufzeichnung des Messsignals über der Zeit bei der Anordnung gemäß Fig. 1 für verschiedene Methankonzentrationen in Wasser,
- Fig. 4: eine Auftragung des relativen Messsignals über der Methankonzentration,
- Fig. 5: den Effekt der Temperatur auf das relative Messsignal,
- Fig. 6: den schematischen Aufbau eines Methansensors als Vorrichtung zur Bestimmung von Kohlenwasserstoffkonzentrationen in Wasser,
- Fig. 7: eine Draufsicht auf ein Leitungssystem zur Zuführung von Wasser zu einer PEM einer Festelektrolytzelle zur Durchführung des Verfahrens,
- Fig. 8: einen Querschnitt durch eine Festelektrolytzelle mit dem Leitungssystem gemäß Fig. 7,
- Fig. 9: einen weiteren Querschnitt durch die Festelektrolytzelle gemäß Fig. 8,
- Fig. 10: den Aufbau des Kerns einer Festelektrolytzelle in einer Draufsicht und
- Fig. 11: einen Querschnitt durch den Kern gemäß Fig. 10.

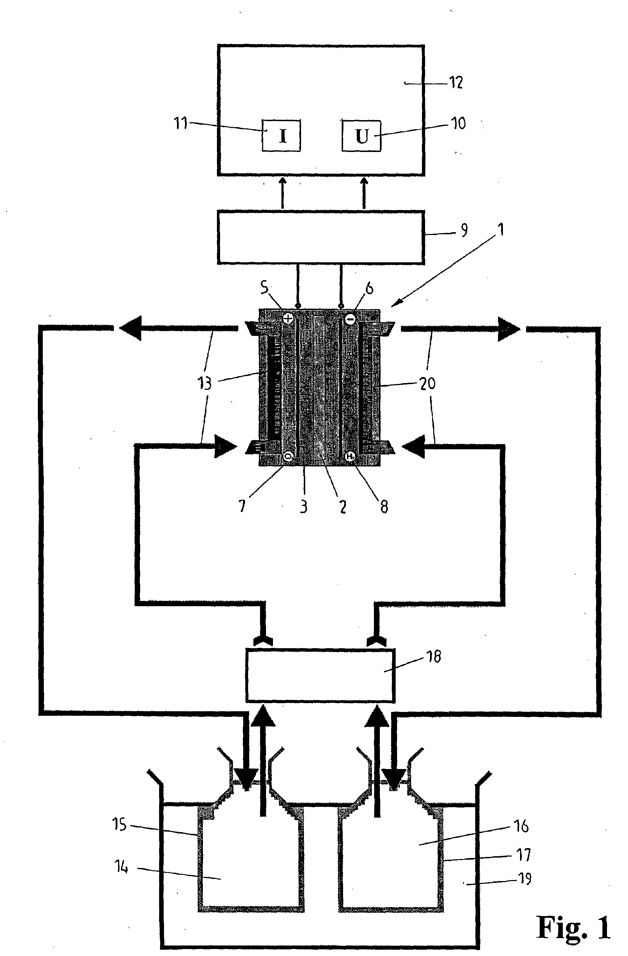

Die in Fig. 1 dargestellte Anordnung zur Durchführung eines Verfahrens zur Bestimmung von Kohlenwasserstoffkonzentrationen in Wasser, insbesondere von Methankonzentrationen in Wasser, umfasst als wesentlichen Bestandteil eine hier schematisch wiedergegebene Festelektrolytzelle 1, die als Hydrolyseur betrieben wird. Festelektrolytzellen dieser Art sind bekannter in ihrer Anwendung als kalte Brennstoffzellen, bei denen nicht wie hier Wasser unter Einsatz elektrischer Energie in Wasserstoff und Sauerstoff aufgespalten wird, sondern Wasserstoff und Sauerstoff zur Gewinnung elektrischer Energie kalt zu Wasser rekombiniert werden. Wesentliches Element der Festelektrolytzelle 1 ist eine Protonenaustauschmembran 2, die aufgrund ihrer englischen Bezeichnung Proton Exchange Membran auch als PEM bezeichnet wird. Die englische Bezeichnung PEM wird auch als Polymerelektrolytmembran interpretiert, wobei diese Interpretation auch hier inhaltlich zutrifft. Konkret kann es sich bei der PEM 2 um eine unter der Marke Nafion von der Firma Du Pont verfügbare Membran handeln. Beiderseits der PEM sind zwei Elektroden 3 und 4 angeordnet, mit denen eine Spannung über die PEM 2 angelegt wird. Hier liegt die Elektrode 3 gegenüber der Elektrode 4 auf positivem Potential, was durch ein Plussymbol 5 und ein Minussymbol 6 angedeutet ist. Entsprechend wird an der Elektrode 3 bei der Elektrolyse von Wasser Sauerstoff 7 freigesetzt, während an der Elektrode 4 Wasserstoff 8 anfällt. Die Spannung zwischen den Elektroden 3 und 4 wird über eine Gleichspannungsquelle 9 angelegt, die die Werte der Gleichspannung 10 und eines durch die Gleichspannung hervorgerufenen Stroms 11 an einen Rechner 12 übermittelt. Auf der Seite der Elektrode 3 wird über ein Leitungssystem 13 Wasser 14 bzw. eine wässrige Lösung herangeführt, die sich in einem Messflüssigkeitsbehälter 15 befindet. Auf der Seite der Elektrode 4 wird über ein Leitungssystem 16 demgegenüber eine wässrige Referenzflüssigkeit 16, bei der es sich um destilliertes Wasser handeln kann, aus einem Referenzflüssigkeitsbehälter 17 herangeführt. Zur Zirkulation der beiden Flüssigkeiten 14 und 16 ist eine Pumpe 18 vorgesehen, die gleiche Volumenströme auf beiden Seiten der PEM 2 in der Festelektrolytzelle 1 bewirkt. Die Flüssigkeitsbehälter 15 und 17 befinden sich in einem Temperierbad 19, um die Temperatur des Wassers 14 und der Referenzflüssigkeit konstant und auf einem gleichen Wert zu halten.

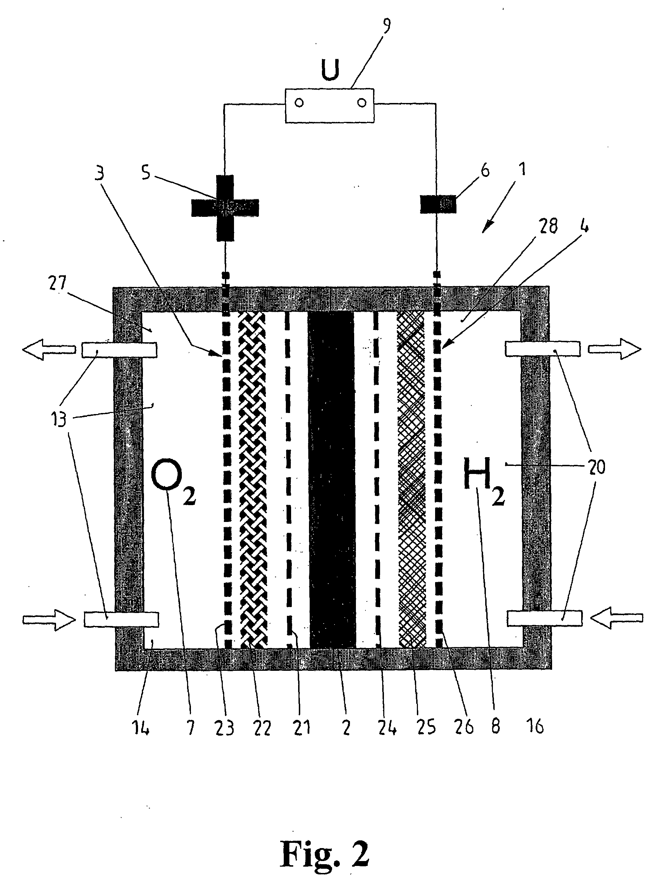

Fig. 2 konzentriert sich in der Darstellung auf die Festelektrolytzelle 1, wobei folgende weitere Details ersichtlich sind. Auf der Sauerstoffseite der PEM 2 ist zunächst eine Katalysatorschicht 21 aus Platinschwarz aufgewalzt. Hieran schließt sich ein offenes blankes Platingitter 22 an. Darüber ist zur Ableitung des Stroms ein gelochtes Titanblech 23 angeordnet. Das Platinschwarz dient als Katalysator. Die Katalysatorschicht 21 kann aber auch schon als erster Bestandteil der Elektrode 3 angesehen werden, die sich dann aus den Teilen 21 bis 23 zusammensetzt. Auf der Wasserstoffseite der PEM 2 ist zunächst eine Gasdiffusionselektrode 24 vorgesehen, die aus Teflon, Kohlenstoff und Platinschwarz zusammengesetzt ist und eine besonders große innere Oberfläche aufweist. Hieran schließt sich ein Platinnetz 25 und dann wieder ein gelochtes Titanblech 26 an. Der Gesamtaufbau der Elektrode 4 beginnt dabei wieder an der Gasdiffusionselektrode 24 und umfasst daher die Teile 24 bis 26. Rückwärtig der Elektroden 3 und 4 sind Räume 27 für das Wasser 14, in dem die Methankonzentration zu bestimmen ist, und 28 für die Referenzflüssigkeit 16 vorgesehen. Das Wasser 14 und die Referenzflüssigkeit 16 dringen von dort über die jeweiligen Elektroden 3 und 4 bis an die PEM vor. Zwischen den Elektroden 3 und 4 wirkt dabei die über die Spannungsquelle 9 angelegte Spannungsdifferenz zwischen den Elektroden 3 und 4 auf die Flüssigkeiten 14 und 16 ein. Beim Überschreiten einer Grenzspannung kommt es zu einer Hydrolyse des Wassers, d. h. zu einer Aufspaltung des Wassers in Sauerstoff 7 und Wasserstoff 8, wobei der Sauerstoff 7 an der Elektrode 3 und der Wasserstoff 8 an der Elektrode 4 freigesetzt wird. Hierzu muss eine Wanderung von Protonen durch die PEM 2 erfolgen. Durch das Vorliegen von Methan oder eines anderen Kohlenwasserstoffs, insbesondere eines anderen kurzkettigen Kohlenwasserstoffs, werden die Eigenschaften der PEM 2 jedoch reversibel so geändert, dass der Protonenstrom durch die PEM 2 und damit der über die PEM 2 fließende elektrische Strom bei konstanter Spannung abnehmen. Zu erklären mag dies über ein kovalente Anbindung der Methanmoleküle an die Oberfläche der PEM 2 sein, wodurch Protonendurchgangswege versperrt werden. Dieser Effekt wird unabhängig von der Polung der Festelektrolytzelle 1 beobachtet und ist damit offensichtlich von dem elektrischen Feld zwischen den Elektroden 3 und 4 aufgrund der angelegten äußeren Spannung weitgehend unabhängig.

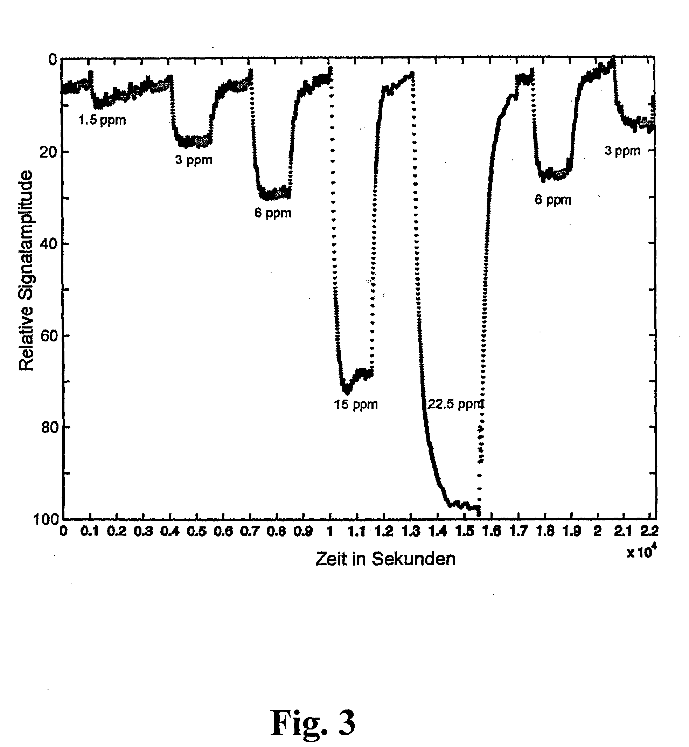

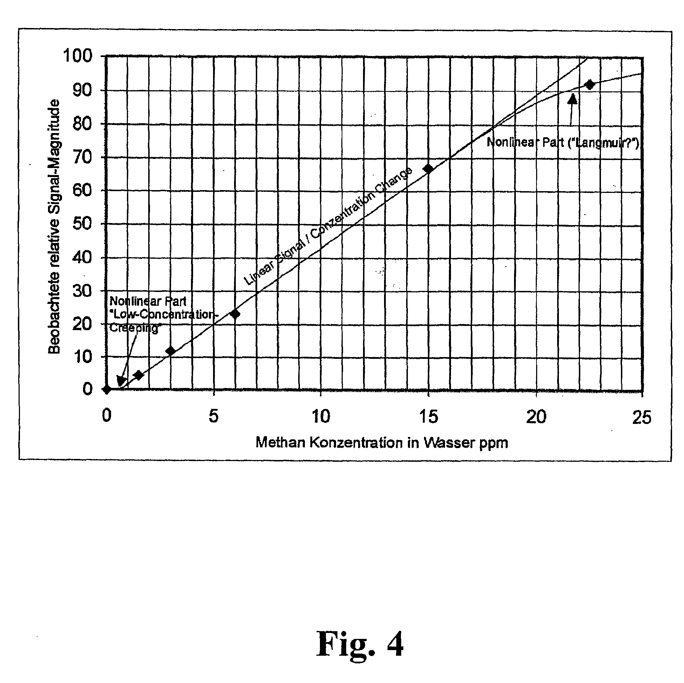

Gleichzeitig ist dieser Effekt der Reduktion der Leitfähigkeit der PEM 2 durch auftretende Methankonzentrationen stark abhängig von der Größe der Methankonzentrationen und er reagiert schnell auf wechselnde Methankonzentrationen. Dies wird anhand der in den Fig. 3 und 4 aufgetragenen Ergebnisse einer Verwendung der Anordnung gemäß den Fig. 1 und 2 im Bezug auf Wasser mit definierten Methankonzentrationen deutlich. Hergestellt wurde eine Ursprungsmethanlösung durch Einleiten von Methan in destilliertes Wasser bei 8 °C unter ständigem Rühren. Anschließendes Erwärmen dieser Lösung auf 25 °C stellte das Erreichen der Sättigungskonzentration für diesen Temperaturwert sicher und verhinderte gleichzeitig Konzentrationsschwankungen im Bereich der Raumtemperatur von 20 °C. Geringere Methankonzentrationen wurden durch Verdünnung dieser gesättigten Ursprungsmethanlösung mit destilliertem Wasser in verschiedenen Verhältnissen erzeugt. Angesetzt wurden auf diese Weise Lösungen mit Methankonzentrationen von 1,5 ppm bis 22,5 ppm. Die entsprechenden Lösungen wurden dann mit 16 ml/min durch die Festelektrolytzelle 1 gemäß Fig. 2 hindurchgepumpt. Mit derselben Geschwindigkeit wurde destilliertes Wasser als Referenzflüssigkeit 16 hindurchgepumpt. Dabei wurde zwischen verschiedenen Messflüssigkeitsbehältern 15, die sich in dem Temperierbad 19 befanden hin- und hergeschaltet. Im Ergebnis ergaben sich die relativen Signale gemäß Fig. 3. Diese Signale sind in der systematisch richtigen Richtung nach unten aufgetragen, weil es sich um abfallende Leitfähigkeiten der PEM 2 handelt. Jede Methankonzentration wird durch ein eindeutiges Messsignal angezeigt, wobei sich die Amplitude des Messsignals über den jeweiligen Messzeitraum nicht wesentlich änderte, sondern schnell einen jeweiligen Endwert erreichte. D. h., das Messsignal spricht in einem Zeitraum von wenigen Minuten vollständig auf die jeweilige Methankonzentration an. Es geht auch über einen Zeitraum von einigen Minuten auf den einer Nullkonzentration in Wasser entsprechenden Ausgangswert zurück, wenn auf destilliertes Wasser ohne Methan umgeschaltet wird. D. h., die dem Messsignal zugrundeliegenden physikalischen Effekte sind reversibel und dies binnen definierter Zeiträume. Zusätzlich sind aber auch methankonzentrationsabhängige dynamische Effekte in dem Verlauf der aufgetragenen Messsignale zu erkennen. D. h., dass auch die Kinetik der Leitfähigkeit in Bezug auf die interessierenden Kohlenwasserstoffkonzentrationen ausgewertet werden kann. Hierbei ist auch die Möglichkeit einer Unterscheidung unterschiedlicher Kohlenwasserstoffe gegeben, da unterschiedliche Kohlenwasserstoffe unter den vorliegenden Messbedingungen allein aufgrund ihrer unterschiedlichen Größe ein unterschiedliches kinetisches Verhalten der resultierenden Leitfähigkeitsreduktionen ergeben.

Fig. 4 ist eine Auftragung der gemessenen relativen Signale über der Methankonzentration in Wasser. Hierbei zeigt sich, dass in einem Bereich von 1,5 bis 15 ppm Methan ein lineares Verhältnis zwischen dem relativen Signal und der Methankonzentration beobachtet werden kann. Bei sehr geringen Methankonzentrationen und sehr hohen Methankonzentrationen machen sich Abweichungen von der Linearität bemerkbar. Diese mögen damit zusammenhängen, dass bei sehr geringen Methankonzentrationen die Behinderung der Protonenwanderung durch die PEM nicht signifikant ist und bei hohen Methankonzentrationen eine Sättigung der Anbindungsplätze für die Methanmoleküle, in der sie eine Behinderung der Protonenwanderung bewirken können, auftritt.

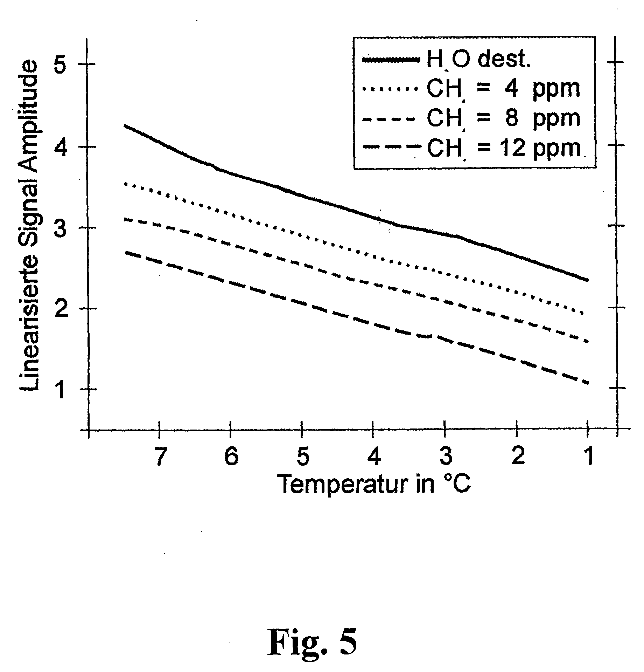

Fig. 5 zeigt den Temperatureffekt der linearen Signalamplitude für verschiedene Methankonzentrationen im Bereich von 1 bis 7,5 °C. Die absoluten Unterschiede der Signalamplitude bleiben über diesen Temperaturbereich für unterschiedliche Methankonzentrationen weitgehend gleich. Bei fallender absoluter Signalamplitude mit fallender Temperatur bedeutet dies, dass die relative Abhängigkeit der Signalamplitude von der Methankonzentration mit fallender Temperatur ansteigt. Es ist also interessant, die Temperatur in dem Temperierbad 19 gemäß Fig. 1 möglichst niedrig zu halten. Die durchgezogene Linie in Fig. 5 steht für destilliertes Wasser, d. h. für eine Konzentration von 0 ppm Methan.

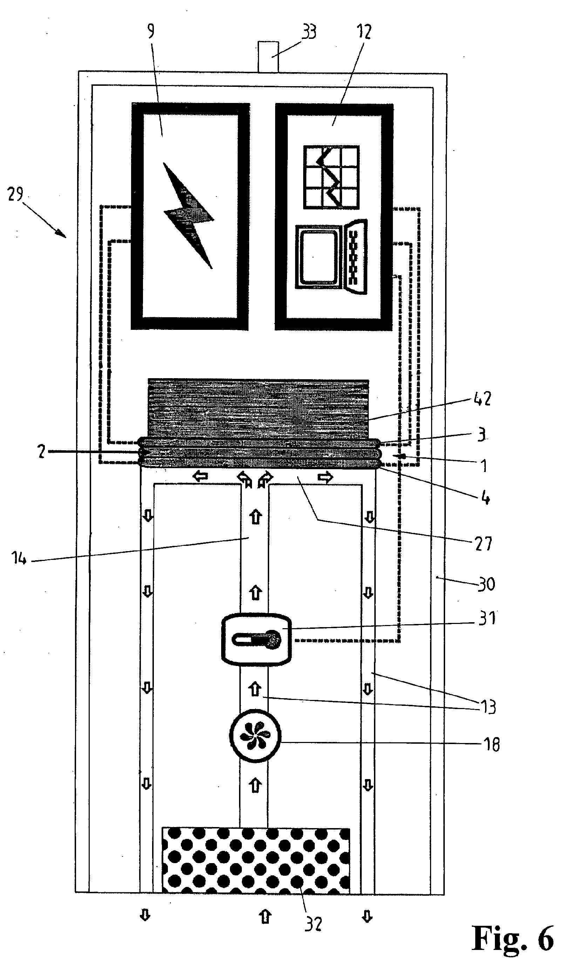

Fig. 6 skizziert einen Methansensor 29 als weitere Vorrichtung zur Umsetzung des hier beschriebenen Verfahrens. In einem Gehäuse 30 ist die Festelektrolytzelle 1 mit der PEM 2 und den Elektroden 3 und 4 angeordnet. Die Elektroden 3 und 4 sind an die hier als Konstantspannungsquelle ausgebildete Spannungsquelle 9 und an den Rechner 12 angeschlossen, der einen weiteren Eingang für einen Temperaturfühler 31 aufweist. Der Temperaturfühler 31 registriert die Temperatur des der Festelektrolytzelle 1 mit der Pumpe 18 zugeführten Wassers 14. Angesaugt wird das Wasser 14 aus der Umgebung des Methansensors 29 über einen Filter 32. Der Filter 32 entfernt Partikelverunreinigung aus dem Wasser 14, was auf die Methankonzentration keinen Einfluss hat. Der Raum 27 für das Wasser liegt hier auf der Seite der Elektrode 4, d. h. der Wasserstoffseite der PEM 2. Gleichzeitig ist der mit einer Referenzflüssigkeit gefüllte Raum auf der anderen Seite der PEM 2 durch eine feste Wasserstoffquelle 42 ersetzt, bei der es sich hier um einen mit Wasserstoff beladenen Metallhydridspeichers handelt. Bei dieser Anordnung erfolgt keine Hydrolyse von Wasser über die PEM 2, sondern zunächst auf der "Sauerstoffseite" der Festelektrolytzelle 1, d.h. der Seite der positiven Elektrode 3 der PEM 2, eine Zerlegung von Wasserstoff unter Abgabe der Elektronen in Protonen, dann eine Leitung der Protonen über die PEM 2 und anschließend auf der Wasserstoffseite an der negativen Elektrode 4 eine Rekombination der Protonen zu Wasserstoff unter Wiederhinzufügung der Elektronen. Bei der Protonenleitung über die PEM 2 machen sich unterschiedliche Methankonzentrationen ebenso bemerkbar wie bei einer Protonenleitung über die PEM 2 im Rahmen der Hydrolyse von Wasser. Der so definierte Methansensor 29 kann über einen analogen und/oder digitalen Port mit einer hier nicht dargestellten Zentraleinheit kommunizieren. Dabei kann unter Verwendung der von dem Temperaturfühler 31 registrierten Temperaturwerte des Wassers 14, dessen Methankonzentration zu bestimmen ist, direkt in dem Methansensor 29 eine Kompensation des Temperatureffekts gemäß Fig. 5 durchgeführt werden.

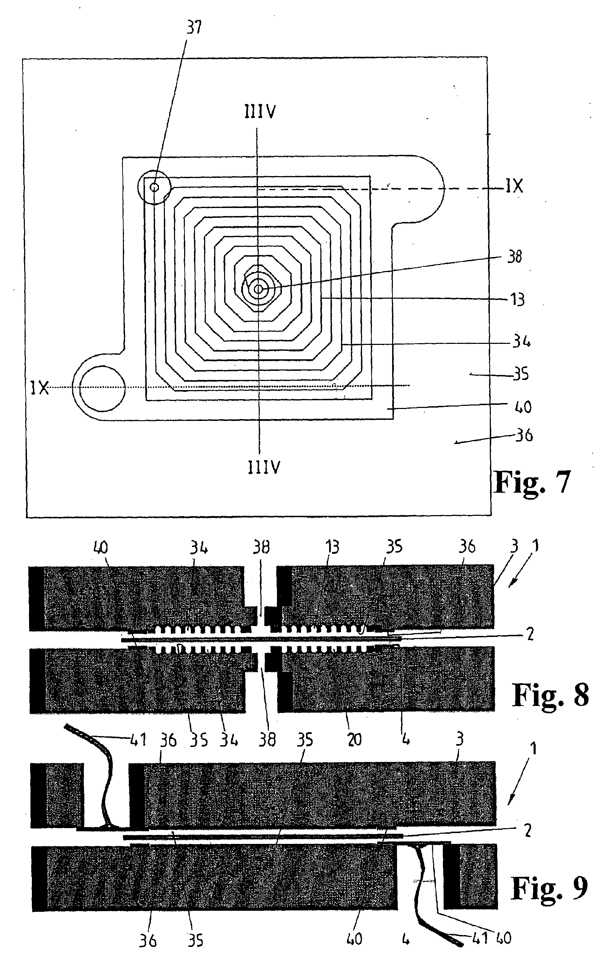

Fig. 7 zeigt das Leitungssystem 13 für das Wasser 14, welches hier als spiralförmiger Kanal 34 in der der PEM zugewandten Oberfläche 35 eine Abdeckplatte 36 der Festelektrolytzelle ausgebildet ist. Der spiralförmige Kanal 34 erstreckt sich von einer senkrecht zu seiner Haupterstreckungsebene verlaufenden Zuführungsbohrung 37 im Randbereich bis zu einer zentralen Abführungsbohrung 38 in der Abdeckplatte 36.

Fig. 8, die einen Schnitt durch den Gesamtaufbau der Festelektrolytzelle 1 mit dem Kanal 34 gemäß Fig. 7 wiedergibt, lässt erkennen, dass die Anordnung der Leitungssysteme 13 und 20 auf beiden Seiten der PEM 2 symmetrisch ist und diese Leitungssysteme 13 und 20 jeweils einen spiralförmigen Kanal 34 und zentrale Abführöffnungen 38 umfassen. Aus Fig. 8 und insbesondere aus Fig. 9, die einen weiteren Schnitt durch die Festelektrolytzelle 1 mit dem Kanal gemäß Fig. 7 zeigt, geht hervor dass jeweils massive Elektrodenrahmen 40 vorgesehen sind, die zur Ausbildung der jeweiligen Elektroden 3 und 4 dienen und eine einfache Kontaktierungsmöglichkeit für Anschlussdrähte 41 schaffen. In der Fläche werden die Elektroden 3 und 4 bei dieser Ausführungsform durch eine hier nicht separat dargestellte Katalysatorschicht aus Platinschwarz auf der PEM 2 ergänzt.

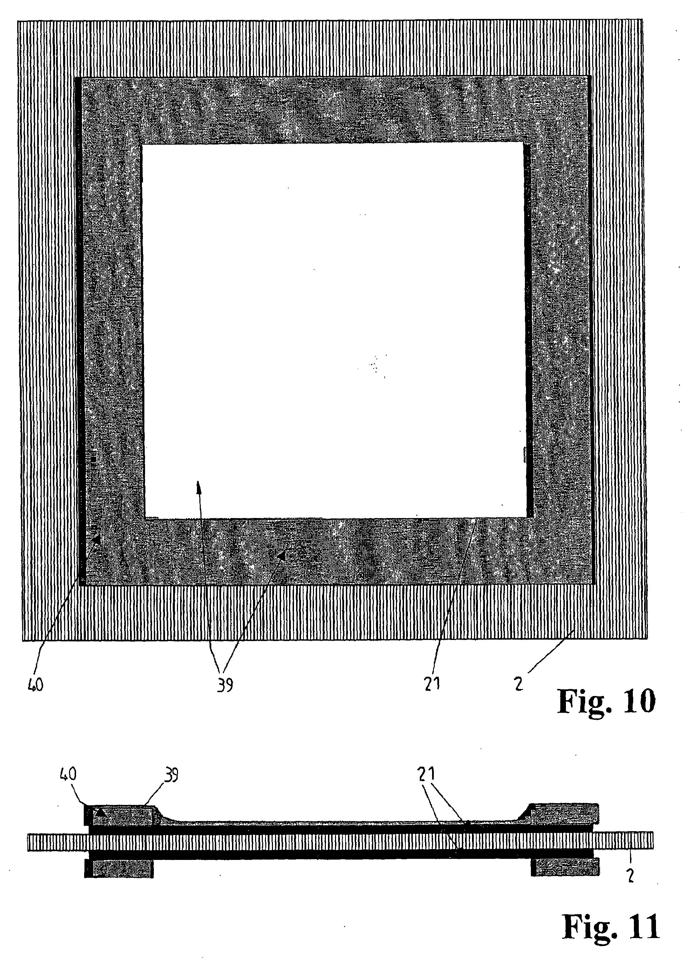

Dies ist auch bei der in den Fig. 10 und 11 gezeigten Ausführungsform des Kernaufbaus einer Festelektrolytzelle 1, d. h. des Schichtaufbaus unmittelbar angrenzend an deren PEM 2 der Fall. Die PEM 2 ist beidseitig mit einer im wesentlichen aus Platinschwarz bestehenden Katalysatorschicht 21 beschichtet. Die Katalysatorschichten 21 werden im Randbereich durch die Elektrodenrahmen 40 überdeckt und kontaktiert. Die Elektrodenrahmen 40 können beispielsweise aus Titan, Platin oder einer Titan/Platinlegierung bestehen. Auf einer Seite ist dann die Anordnung oberhalb des Elektrodenrahmens 40 bzw. der Katalysatorschicht 21 mit einer Silikonmembran 39 als Gasdiffusionsschicht abgedeckt. Die Silikonmembran 39 hat die primäre Aufgabe, die PEM 2, die Katalysatorschicht 21 aus Platinschwarz und den Elektrodenrahmen 40 vor Substanzen zu schützen, die in dem Wasser, in dem die Methankonzentration zu messen ist, enthalten sein können. Sie läßt zu diesem Zweck beispielsweise keine Ionen aus Salzwasser zu der PEM 2 hindurchtreten. Methan kann jedoch ohne weiteres durch die Silikonmembran 39 gelangen. Dabei ist es aber nicht vorgesehen, dass zwischen der Silikonmembran 39 und der PEM 2 über die Katalysatorschicht 21 Platinschwarz ein Gasraum ausgebildet wird. Vielmehr wird dieser Raum durch Osmose durch die Polymermembran 39 nass gehalten. Bei dem Messen der Methankonzentration in Salzwasser ist neben der Silikonmembran 39 auf der dem Salzwasser zugewandten Seite der PEM-Membran 2 sinnvoll, das Salzwasser auf der Wasserstoffseite der PEM-Membran heranzuführen, weil auf der Sauerstoffseite eine freie Platinoberfläche als Katalysator für die definierte Funktion der Festelektrolytzelle als Hydrolyseur vorliegen muss, die Gefahr läuft, nascierendes Chlor, das bei der Hydrolyse von Salzwasser an ihrer Oberfläche entsteht, angegriffen und über die Zeit verändert zu werden, was zumindest die Langzeitstabilität der Signale eines Methansensors gefährden würde.

### BEZUGSZEICHENLISTE

- 1 -: Festelektrolytzelle
- 2 -: Protonenaustauschmembran (PEM)
- 3 -: Elektrode
- 4 -: Elektrode
- 5 -: Plussymbol
- 6 -: Minussymbol
- 7 -: O₂
- 8-: H₂
- 9 -: Spannungsquelle
- 10 -: Spannung
- 11 -: Strom
- 12 -: Rechner
- 13 -: Leitungssystem
- 14 -: Wasser
- 15 -: Messflüssigkeitsbehälter
- 16 -: Leitungssystem
- 17 -: Referenzflüssigkeitsbehälter
- 18 -: Pumpe
- 19 -: Temperierbad
- 20 -: Leitungssystem
- 21 -: Katalysatorschicht
- 22 -: Platingitter
- 23 -: Titanblech
- 24 -: Gasdiffusionselektrode
- 25 -: Platingitter
- 26 -: Titanblech
- 27 -: Raum
- 28 -: Raum
- 29 -: Methansensor
- 30 -: Gehäuse
- 31 -: Temperaturfühler
- 32 -: Filter
- 33 -: Port
- 34 -: Kanal
- 35 -: Abdeckplatte
- 36 -: Oberfläche
- 37 -: Zuführbohrung
- 38 -: Abführbohrung
- 39 -: Silikonmembran
- 40 -: Elektrodenrahmen
- 41 -: Anschlussdrähte
- 42 -: Wasserstoffquelle

## Patentansprüche

1. Verfahren zur Bestimmung einer Kohlenwasserstoffkonzentration, insbesondere einer Methankonzentration, in Wasser, wobei eine von der Kohlenwasserstoffkonzentration abhängige Leitfähigkeit bestimmt wird, **dadurch gekennzeichnet, dass** die Leitfähigkeit über eine Protonenaustauschmembran (PEM) (2) bestimmt wird.

2. Verfahren nach Anspruch 1, **dadurch gekennzeichnet, dass** auf der einen Seite der PEM (2) das Wasser (14), in dem die Kohlenwasserstoffkonzentration zu bestimmen ist, und auf der andere Seite der PEM (2) eine Referenzsubstanz (16, 42) angeordnet wird.

3. Verfahren nach Anspruch 2, **dadurch gekennzeichnet, dass** die Referenzsubstanz eine wässrige Referenzflüssigkeit (16) ist.

4. Verfahren nach Anspruch 2 oder 3, **dadurch gekennzeichnet, dass** das Wasser (14) und/oder eine flüssige Referenzsubstanz (16) zu einer Strömung über die Fläche der jeweiligen Seite der PEM (2) gezwungen wird.

5. Verfahren nach einem der Ansprüche 1 bis 4, **dadurch gekennzeichnet, dass** eine feste Spannung (10) über die PEM (2) angelegt wird und dass der bei dieser Spannung (10) fließende Strom (11) über die PEM (2) gemessen wird.

6. Verfahren nach einem der Ansprüche 2 bis 4, **dadurch gekennzeichnet, dass** eine Gleichspannung über die PEM (2) angelegt wird, wobei das positive oder das negative Potential auf der Seite der PEM (2) angelegt wird, auf der das Wasser (14), in dem die Kohlenwasserstoffkonzentration zu bestimmen ist, angeordnet wird.

7. Verfahren nach einem der Ansprüche 1 bis 5, **dadurch gekennzeichnet, dass** eine Wechselspannung über die PEM (2) angelegt wird.

8. Verfahren nach einem der Ansprüche 2 bis 4, **dadurch gekennzeichnet, dass** auf der Seite der PEM (2), auf der das Wasser (14), in dem die Kohlenwasserstoffkonzentration zu bestimmen ist, angeordnet wird, eine massiver Elektrodenrahmen (40) im Randbereich der PEM (2) angeordnet und mit einer leitenden Katalysatorschicht (21) auf der PEM (2) kontaktiert wird.

9. Verfahren nach einem der Ansprüche 2 bis 4, **dadurch gekennzeichnet, dass** auf der Seite der PEM (2), auf der das Wasser (14), in dem die Kohlenwasserstoffkonzentration zu bestimmen ist, angeordnet wird, eine Silikonmembran (39) vor der PEM (2) angeordnet wird.

10. Verfahren nach einem der Ansprüche 1 bis 9, **dadurch gekennzeichnet, dass** die Parameter des Wassers (14) neben der zu bestimmenden Kohlenwasserstoffkonzentration auf vorgegebene Werte eingestellt und/oder bestimmt werden.

11. Vorrichtung zur Bestimmung einer Kohlenwasserstoffkonzentration, insbesondere einer Methankonzentration, in Wasser, mit einer Messanordnung, deren Leitfähigkeit von der Kohlenwasserstoffkonzentration abhängig ist, **dadurch gekennzeichnet, dass** die Messanordnung eine Protonenaustauschmembran (PEM) (2) aufweist.

12. Vorrichtung nach Anspruch 11, **dadurch gekennzeichnet, dass** die PEM (2) eine Polymerelektrolytmembran ist.

13. Vorrichtung nach Anspruch 11 oder 12, **dadurch gekennzeichnet, dass** auf der einen Seite der PEM (2) ein Raum (27) für das Wasser (14), in dem die Kohlenwasserstoffkonzentration zu bestimmen ist, und auf der andere Seite der PEM (2) eine Referenzsubstanz (16, 42) angeordnet ist.

14. Vorrichtung nach Anspruch 13, **dadurch gekennzeichnet, dass** der Raum (27) für das Wasser (14) und/oder ein Raum (28) für eine flüssige Referenzsubstanz (16) ein über die Fläche der jeweiligen Seite der PEM (2) führendes Leitungssystem (13 bzw. 20) aufweist, wobei eine Pumpe (18) vorgesehen ist, die das Wasser (14) bzw. die flüssige Referenzsubstanz (16) durch das Leitungssystem (13 bzw. 20) hindurchpumpt.

15. Vorrichtung nach einem der Ansprüche 11 bis 14, **dadurch gekennzeichnet, dass** eine Spannungsquelle (9) vorgesehen ist, die eine feste Spannung (10) über die PEM (2) abgibt, und dass ein Amperemeter vorgesehen ist, das den bei dieser Spannung (10) fließende Strom (11) über die PEM (2) misst.

16. Vorrichtung nach einem der Ansprüche 13 und 14, **dadurch gekennzeichnet, dass** eine Gleichspannungsquelle vorgesehen ist, die eine Gleichspannung über die PEM (2) abgibt, wobei das positive oder das negative Potential auf der Seite der PEM (2) anliegt, auf der der Raum (27) für das Wasser (14), in dem die Kohlenwasserstoffkonzentration zu bestimmen ist, angeordnet ist.

17. Vorrichtung nach einem der Ansprüche 11 bis 15, **dadurch gekennzeichnet, dass** eine Wechselspannungsquelle vorgesehen ist, die eine Wechselspannung über die PEM (2) abgibt.

18. Vorrichtung nach einem der Ansprüche 13 und 4, **dadurch gekennzeichnet, dass** auf der Seite der PEM (2), auf der der Raum (27) für das Wasser (14), in dem die Kohlenwasserstoffkonzentration zu bestimmen ist, angeordnet wird, ein massiver Elektrodenrahmen (40) im Randbereich der PEM (2) angeordnet und mit einer leitenden Katalysatorschicht (21) auf der PEM (2) kontaktiert ist.

19. Vorrichtung nach einem der Ansprüche 13 und 14, **dadurch gekennzeichnet, dass** zwischen dem Raum (28) für das Wasser (14), in dem die Kohlenwasserstoffkonzentration zu bestimmen ist, und der PEM (2) eine Silikonmembran (39) angeordnet ist.

20. Verfahren nach einem der Ansprüche 11 bis 19, **dadurch gekennzeichnet, dass** Konditionier- und/oder Messeinrichtungen (31) vorgesehen sind, um die Parameter des Wassers (14) neben der zu bestimmenden Kohlenwasserstoffkonzentration auf vorgegebene Werte einzustellen und/oder zu bestimmen.
